(19) Europäisches Patentamt
European Patent Office
Office européen des brevets

(11) Veröffentlichungsnummer: **0 622 369 A2**

(12) **EUROPÄISCHE PATENTANMELDUNG**

(21) Anmeldenummer: **94105779.6**

(22) Anmeldetag: **14.04.94**

(51) Int. Cl.$^{5}$: **C07D 491/048**, C07D 401/10, A61K 31/47

(30) Priorität: **27.04.93 DE 4313693**

(43) Veröffentlichungstag der Anmeldung: **02.11.94 Patentblatt 94/44**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Stoltefuss, Jürgen, Dipl.-Ing.**
**Parkstrasse 20**
**D-42781 Haan (DE)**
Erfinder: **Goldmann, Siegfried, Dr.**
**Am Osterholz 91**
**D-42327 Wuppertal (DE)**
Erfinder: **Straub, Alexander, Dr.**
**Moospfad 30**
**D-42113 Wuppertal (DE)**
Erfinder: **Bechem, Martin, Dr.**
**Hans-Böckler-Strasse 102**
**D-42111 Wuppertal (DE)**
Erfinder: **Gross, Rainer, Prof. Dr.**
**Platzhofstrasse 102**
**D-42111 Wuppertal (DE)**
Erfinder: **Hebisch, Siegbert, Dr.**
**Johann-Breuker-Platz 8**
**D-46244 Bottrop (DE)**
Erfinder: **Hütter, Joachim, Dr.**
**Teschen-Sudberger-Strasse 13**
**D-42349 Wuppertal (DE)**
Erfinder: **Rounding, Howard-Paul, Dr.**
**Pahlkestrasse 15**
**D-42115 Wuppertal (DE)**

(54) **2-Amino-4-chinolin-dihydropyridine, Verfahren zu ihrer Herstellung und ihre Verwendung.**

(57) Die vorliegende Erfindung betrifft 2-Amino-4-chinolin-1,4-dihydropyridine der allgemeinen Formel (I)

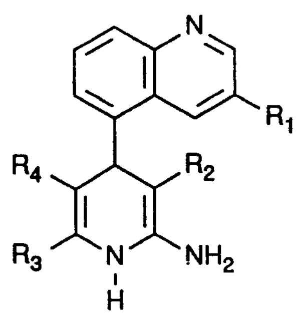

(I)

in welcher $R_1$ bis $R_4$ die in der Beschreibung angegebene Bedeutung haben, Verfahren zu ihrer Herstellung und ihre Verwendung in Arzneimitteln, insbesondere in Mitteln zur Behandlung von Herz-Kreislauferkrankungen.

EP 0 622 369 A2

Die vorliegende Erfindung betrifft neue 2-Amino-4-chinolin-1,4-dihydropyridine, Verfahren zu ihrer Herstellung und ihre Verwendung in Arzneimitteln, insbesondere in Mitteln zur Behandlung von Herz-Kreislauferkrankungen.

Es ist bereits bekannt, daß einige 2- und 6-Amino-3,4-dihydropyridine neben einer antiarrhythmischen auch eine lipidabsorptionshemmende Wirkung besitzen [vgl. EP 73 997]. Es sind auch schon 2-Amino-1,4-dihydropyridine mit einer vasodilatorischen und antihypertensiven Wirkung beschrieben. Außerdem werden einige der erfindungsgemäßen Verbindungen der Formel (I) durch die allgemein sehr breiten Definitionen in EP 71 819 umfaßt, ohne daß dort jedoch entsprechende konkrete Stoffe genannt sind.

Die vorliegende Erfindung betrifft 2-Amino-4-chinolin-1,4-dihydropyridine der allgemeinen Formel (I)

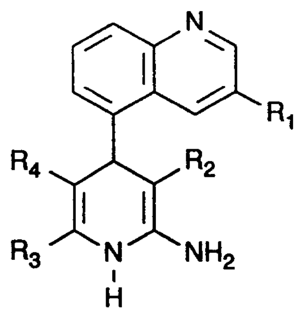

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio, geradkettiges oder verzweigtes Alkoxy oder Alkoxycarbonyl mit jeweils bis zu 8 Kohlenstoffatomen, Carboxy, Amino, Dimethylamino oder Hydroxy
oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder
für Thienyl oder Pyridyl steht, die gegebenenfalls durch Halogen substituiert sind,

$R^2$ für eine Gruppe der Formel $-CO-NR^5R^6$ oder $-CO-A-R^7$ steht,
worin

$R^5$ und $R^6$ gleich oder verschieden sind und Wasserstoff einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist,
wobei die Carbo- und Hetero-Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,
oder

$R^5$ und $R^6$ gemeinsam unter Einbezug des Stickstoffatoms einen 3- bis 8-gliedrigen, gesättigen oder ungesättigten Heterocyclus bilden, der gegebenenfalls durch ein Sauerstoffatom oder durch einen Rest der Formel $S(O)_a$, -CO- oder $-NR^8$ unterbrochen sein kann,
worin

a eine Zahl 0, 1 oder 2 bedeutet,

$R^8$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder
einen cyclischen, gesättigen oder ungesättigen, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkxoy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

A eine direkte Bindung oder ein Sauerstoffatom bedeutet,

$R^7$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder
einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_b-$ oder $-NR^9$ unterbrochen ist,
worin

b die oben angegebene Bedeutung von a hat und mit dieser gleich oder verschieden ist,

$R^9$ die oben angegebene Bedeutung von $R^8$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

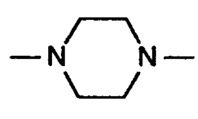 , 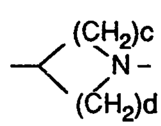 , 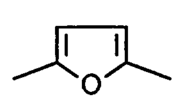 oder 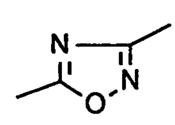

unterbrochen ist,
worin

c und d gleich oder verschieden sind und eine Zahl 1 oder 2 bedeuten,

und worin Aryliden seinerseits durch Halogen. Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, die ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

durch eine Gruppe der Formel $-CO_2-R^{10}$, $-CONR^{11}R^{12}$ oder $-NR^{13}R^{14}$ substituiert ist,

worin

$R^{10}$ die oben angegebene Bedeutung von $R^8$ hat und mit dieser gleich oder verschieden ist

und

$R^{11}$, $R^{12}$, $R^{13}$ und $R^{14}$ die oben angegebene Bedeutung von $R^5$ und $R^6$ haben und mit diesen gleich oder verschieden sind,

$R^3$ für Wasserstoff oder geradkettiges oder verzweigtes Alkyl mit bis zu 8 Kohlenstoffatomen steht,

$R^4$ für Nitro oder Formyl steht,

oder

$R^3$ und $R^4$ zusammen einen Lactonring der Formel

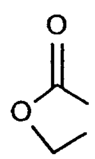

bilden,

und deren Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),

in welcher

$R^1$ für Phenyl steht, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Carboxy, Amino, Dimethylamino oder Hydroxy substituiert ist, oder

für Thienyl oder Pyridyl steht, die gegebenenfalls durch Fluor, Chlor oder Brom substituiert sind,

$R^2$ für eine Gruppe der Formel $-CO-NR^5R^6$ oder $-CO-A-R^7$ steht,

worin

$R^5$ und $R^6$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor

oder durch Phenyl substituiert ist, das seinerseits durch Fluor, Chlor oder durch geradkettiges oder verzweigtes Alkyl, Alkoxy oder Alkylthio mit jeweils bis zu 4 Kohlenstoffatomen, Trifluormethyl oder Trifluormethoxy substituiert sein kann, oder

Phenyl bedeuten, das gegebenenfalls durch Fluor, Chlor, Brom, Cyano oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert ist,

oder

$R^5$ und $R^6$ gemeinsam unter Einbezug des Stickstoffatoms einen 5- bis 6-gliedrigen, gesättigen oder ungesättigten Heterocyclus bilden, der gegebenenfalls durch einen Rest der Formel $-NR^8$ unterbrochen sein kann,

worin

$R^8$ Wasserstoff oder Phenyl bedeutet, das gegebenenfalls durch Fluor, Chlor, Brom, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert ist, oder

einen cyclischen, gesättigten oder ungesättigten, geradkettigen oder verzweigten Kohlenwasserstoffrest mit bis zu 6 Kohlenstoffatomen bedeutet, der gegebenenfalls durch Hydroxy, Fluor, Chlor, Brom oder durch Phenyl oder Pyridyl substituiert ist, die ihrerseits bis zu 2-fach gleich oder verschieden durch Fluor, Chlor, Brom, Cyano oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sein können,

A eine direkte Bindung oder ein Sauerstoffatom bedeutet,

$R^7$ Wasserstoff oder Phenyl oder Pyridyl bedeutet, die gegebenenfalls durch Fluor, Chlor oder durch geradkettiges oder verzweigtes Alkyl, Alkoxy oder Alkylthio mit jeweils bis zu 4 Kohlenstoffatomen, Trifluormethyl oder Trifluormethoxy substituiert sind, oder einen cyclischen, geradkettigen oder verzweigten, gesättigten oder ungesättigten Kohlenwasserstoffrest mit bis zu 8 Kohlenstoffatomen bedeutet, der gegebenerfalls bis zu 2-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_b-$ oder $-NR^9$ unterbrochen ist,

worin

b eine Zahl 0, 1 oder 2 bedeutet,

$R^9$ die oben angegebene Bedeutung von $R^8$ hat und mit dieser gleich oder verschieden ist,

oder der Kohlenwasserstoffrest gegebenenfalls bis zu 2-fach gleich oder verschieden durch Phenyliden oder heterocyclische Reste der Formeln

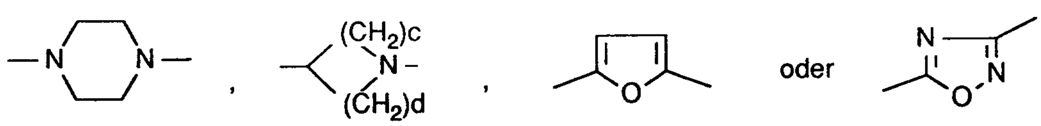

unterbrochen ist,

worin

c und d gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl, Cyclopentyl, Cyclohexyl, Fluor, Chlor, Brom, Nitro, Cyano, Hydroxy, $-O-NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, die ihrerseits durch Fluor, Chlor oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sein können, oder

durch eine Gruppe der Formel $-CO_2-R^{10}$, $-CONR^{11}R^{12}$ oder $-NR^{13}R^{14}$ substitu-

iert ist,
worin

$R^{10}$ — die oben angegebene Bedeutung von $R^8$ hat und mit dieser gleich oder verschieden ist
und

$R^{11}$, $R^{12}$, $R^{13}$ und $R^{14}$ — die oben angegebene Bedeutung von $R^5$ und $R^6$ haben und mit diesen gleich oder verschieden sind,

$R^3$ — für Wasserstoff oder geradkettiges oder verzweigtes Alkyl mit bis zu 4 Kohlenstoffatomen steht,

$R^4$ — für Nitro oder Formyl steht,
oder

$R^3$ und $R^4$ — zusammen einen Lactonring der Formel

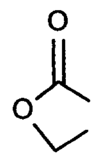

bilden,

und deren Salze.

Besonders bevorzugt sind Verbindungen der allgemeinen Formel (I)
in welcher

$R^1$ — für Phenyl steht, das gegebenenfalls bis zu 2-fach gleich oder verschieden durch Fluor, Chlor, Nitro, Trifluormethyl, Methyl, Ethyl, Methoxy, Ethoxy, Dimethylamino oder Hydroxy substituiert ist, oder
für Thienyl oder Pyridyl steht,

$R^2$ — für eine Gruppe der Formel $-CO-NR^5R^6$ oder $-CO-A-R^7$ steht,
worin

$R^5$ und $R^6$ — gleich oder verschieden sind und Wasserstoff oder einen geradkettigen, verzweigten, cyclischen, gesättigten oder ungesättigten Kohlenwasserstoffrest mit bis zu 5 Kohlenstoffatomen bedeuten, der gegebenenfalls durch Fluor, Chlor oder durch Phenyl substituiert ist, das seinerseits durch Fluor, Chlor, Methyl, Methoxy, Methylthio, Trifluormethyl oder Trifluormethoxy substituiert sein kann,
oder
Phenyl bedeuten, das gegebenenfalls durch Fluor, Chlor oder durch Methyl, Methoxy, Trifluormethyl oder Trifluormethoxy substituiert ist,
oder

$R^5$ und $R^6$ — gemeinsam unter Einbezug des Stickstoffatoms einen 5- bis 7-gliedrigen, gesättigen oder ungesättigten Heterocyclus bilden, der gegebenenfalls durch ein Sauerstoffatom oder durch einen Rest der Formel $-NR^8$ unterbrochen sein kann,
worin

$R^8$ — Wasserstoff oder Phenyl bedeutet, oder
einen cyclischen, gesättigten oder ungesättigten, geradkettigen oder verzweigten Kohlenwasserstoffrest mit bis zu 4 Kohlenstoffatomen bedeutet, der gegebenenfalls durch Fluor, Chlor, Brom oder durch Phenyl substituiert ist,

A — eine direkte Bindung oder ein Sauerstoffatom bedeutet,

$R^7$ — Wasserstoff oder Phenyl bedeutet, oder
einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder durch -CO-, -CO-NH-, -O-CO-, -CO-O-, -NH-CO-, $-SO_2-NH-$, $-NH-SO_2-$, $-S(O)_b-$ oder $-NR^9$ unterbrochen ist,
worin

b — eine Zahl 0,1 oder 2 bedeutet,

$R^9$ — die oben angegebene Bedeutung von $R^8$ hat und mit dieser gleich oder verschieden ist,

oder der Kohlenwasserstoffrest bis zu 2-fach gleich oder verschieden durch Phenyliden oder heterocyclische Reste der Formeln

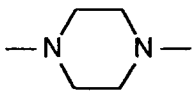 , 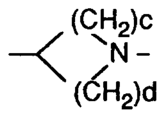 , 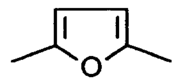 oder 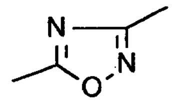

unterbrochen ist,
worin

c und d gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl, Cyclopentyl, Cyclohexyl, Fluor, Chlor, Nitro, Cyano, Hydroxy, $-O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, die ihrerseits durch Fluor, Chlor oder durch Alkyl, Alkoxy, Alkylthio, Alkoxycarbonyl mit jeweils bis zu 2 Kohlenstoffatomen, Trifluormethyl oder Trifluormethoxy substituiert sein können, oder

durch eine Gruppe der Formel $-CO_2-R^{10}$, $-CONR^{11}R^{12}$ oder $-NR^{13}R^{14}$ substituiert ist,
worin

$R^{10}$ die oben angegebene Bedeutung von $R^8$ hat und mit dieser gleich oder verschieden ist
und

$R^{11}$, $R^{12}$, $R^{13}$ und $R^{14}$ die oben angegebene Bedeutung von $R^5$ und $R^6$ haben und mit diesen gleich oder verschieden sind,

$R^3$ für Wasserstoff, Methyl oder Ethyl steht,

$R^4$ für Nitro oder Formyl steht,
oder

$R^3$ und $R^4$ zusammen einen Lactonring der Formel

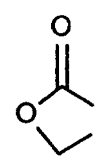

bilden,

und deren Salze.

Ganz besonders bevorzugt sind Verbindungen der allgemeinen Formel (I),
in welcher

$R^1$ für Phenyl steht, das gegebenenfalls durch Fluor, Chlor, Methyl oder Methoxy substituiert ist,

$R^2$ für eine Gruppe der Formel $-CO-A-R^7$ steht,
worin

A ein Sauerstoffatom bedeutet,

$R^7$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff, Schwefel, $SO_2$, -CO-NH-, -NH-CO-, -CO-O unterbrochen ist, und der gegebenenfalls durch Cyclopropyl, Cyclopentyl, Cyclohexyl,
Fluor, Chlor oder durch Phenyl, Phenoxy oder Phenylthio substituiert ist, wobei die Cyclen ihrerseits durch Fluor, Chlor, Methyl oder Methoxy substituiert sein können,

$R^3$ für Wasserstoff oder Methyl steht,

$R^4$ für Nitro steht,

oder

$R^3$ und $R^4$ zusammen einen Lactonring der Formel

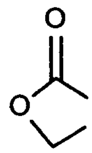

bilden,

und deren Salze.

Die Herstellung der erfindungsgemäßen Verbindungen der allgemeinen Formel (I), ist dadurch gekennzeichnet, daß man

[A] entweder Aldehyde der allgemeinen Formel (II)

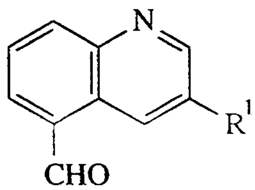

(II)

in welcher

$R^1$ die oben angegebene Bedeutung hat,

direkt mit Verbindungen der allgemeinen Formel (III)

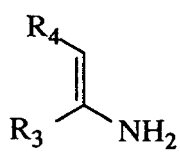

(III)

in welcher

$R^3$ und $R^4$ die oben angegebene Bedeutung haben,

und Verbindungen der tautomeren Formeln (IV) und (IVa)

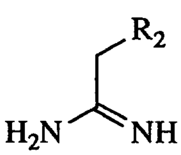

(IV)

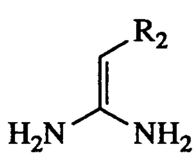

(IVa)

in welcher

$R^2$ die oben angegebene Bedeutung hat,

in inerten Lösemitteln bei Temperaturen zwischen 10 °C und 150 °C umsetzt,

oder

[B] Verbindungen der allgemeinen Formel (II) mit Verbindungen der allgemeinen Formel (V)

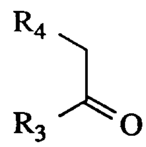

(V)

in welcher

$R^3$ und $R^4$ die oben angegebene Bedeutung haben,

gegebenenfalls nach Isolierung der Ylidenverbindungen der allgemeinen Formel (VI)

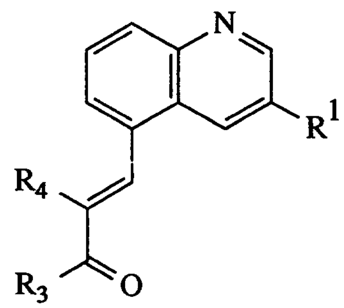

(VI)

in welcher

$R^1$, $R^3$ und $R^4$ die oben angegebene Bedeutung haben,

mit Verbindungen der allgemeinen Formel (VII) bzw. (VIIa)

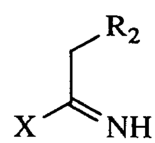

(VII)

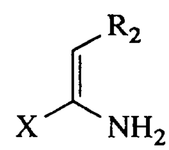

(VIIa)

in welcher

$R^2$ die oben angegebene Bedeutung hat, und

X für die Aminogruppe oder für $C_1$-$C_4$-Alkoxy steht,

gegebenenfalls in Anwesenheit von inerten organischen Lösemitteln bei Temperaturen von 10 °C bis 150 °C umsetzt, wobei für den Fall, daß X für $C_1$-$C_4$-Alkoxy steht, Ammoniumsalze, wie Ammoniumacetat zugegeben werden,

oder im Fall, daß $R^3$ und $R^4$ gemeinsam einen Lactonring bilden

[C] zunächst nach denen unter [A] und [B] aufgeführten Methoden, Verbindungen der allgemeinen Formel (VIII)

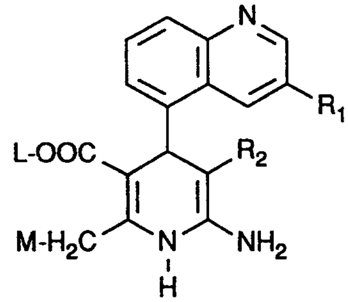

(VIII)

in welcher

| | |
|---|---|
| $R^1$, $R^2$ und $R^3$ | die oben angegebene Bedeutung haben, |
| L | für einen $C_1$-$C_8$-Alkyl-Rest steht und |
| M | für eine Abgangsgruppe wie beispielsweise Chlor oder Acetoxy steht, |

herstellt, und nach bekannten Verfahren einen säure- oder basenkatalysierten Ringschluß anschließt.

Im Fall der reinen Enantiomeren wird entweder das entstehende Diastereomerengemisch der jeweiligen Verbindungen der allgemeinen Formel (I), in welcher $R^2$ für einen definierten chiralen Rest steht, zunächst getrennt, dann in die entsprechenden Carbonsäuren ($R^{2'}$ = $CO_2H$) überführt und in einem letzten Schritt verestert oder die jeweiligen Diastereomere werden direkt mit den entsprechenden Alkoholen, insbesondere in Form der Alkoholate umgeestert

Die erfindungsgemäßen Verfahren können durch folgendes Formelschema beispielhaft erläutert werden:

[A]

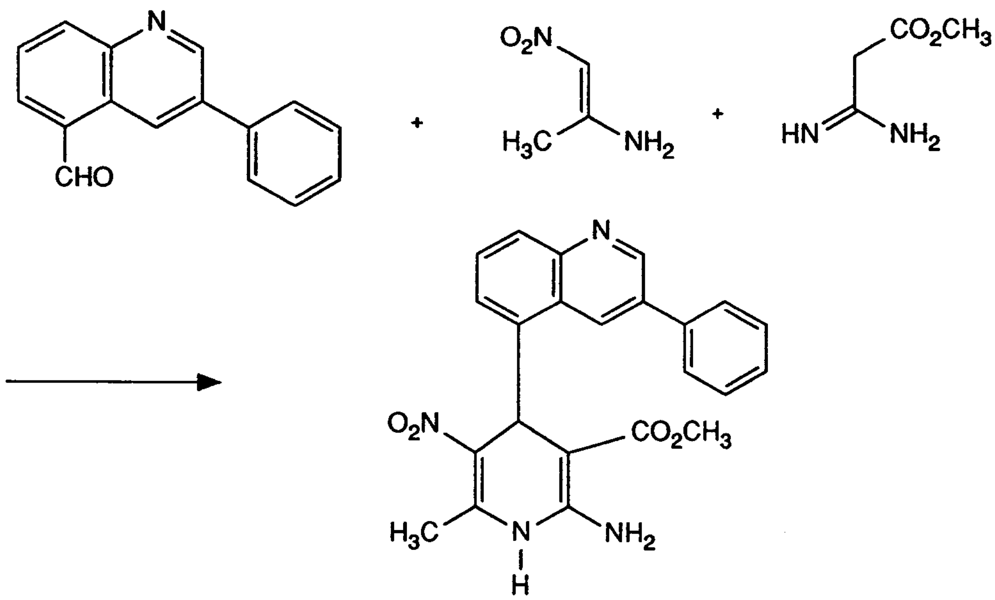

[B]

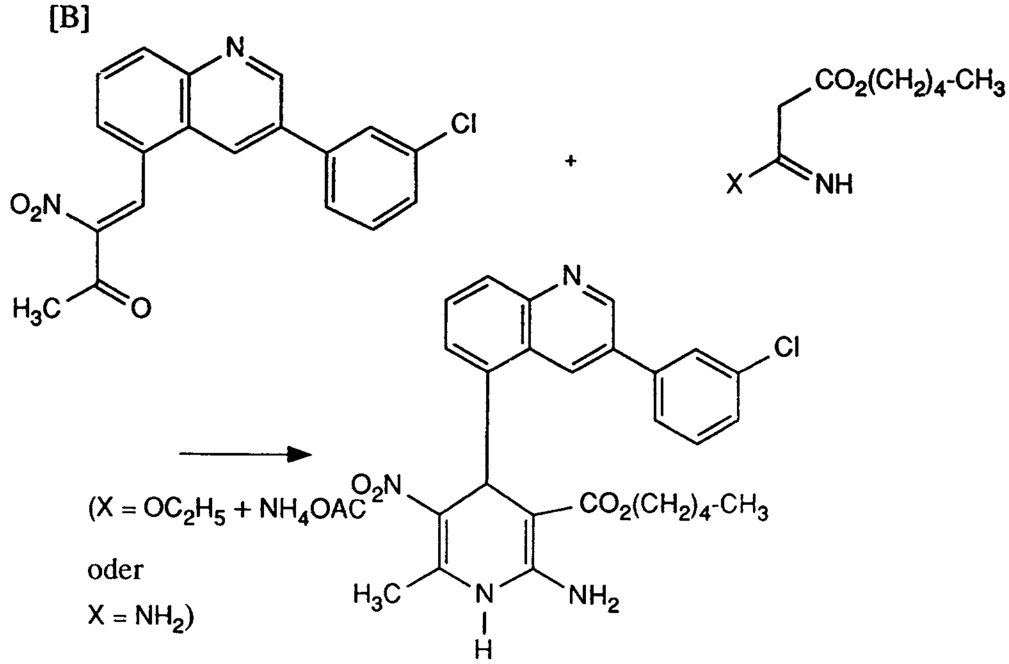

[C]

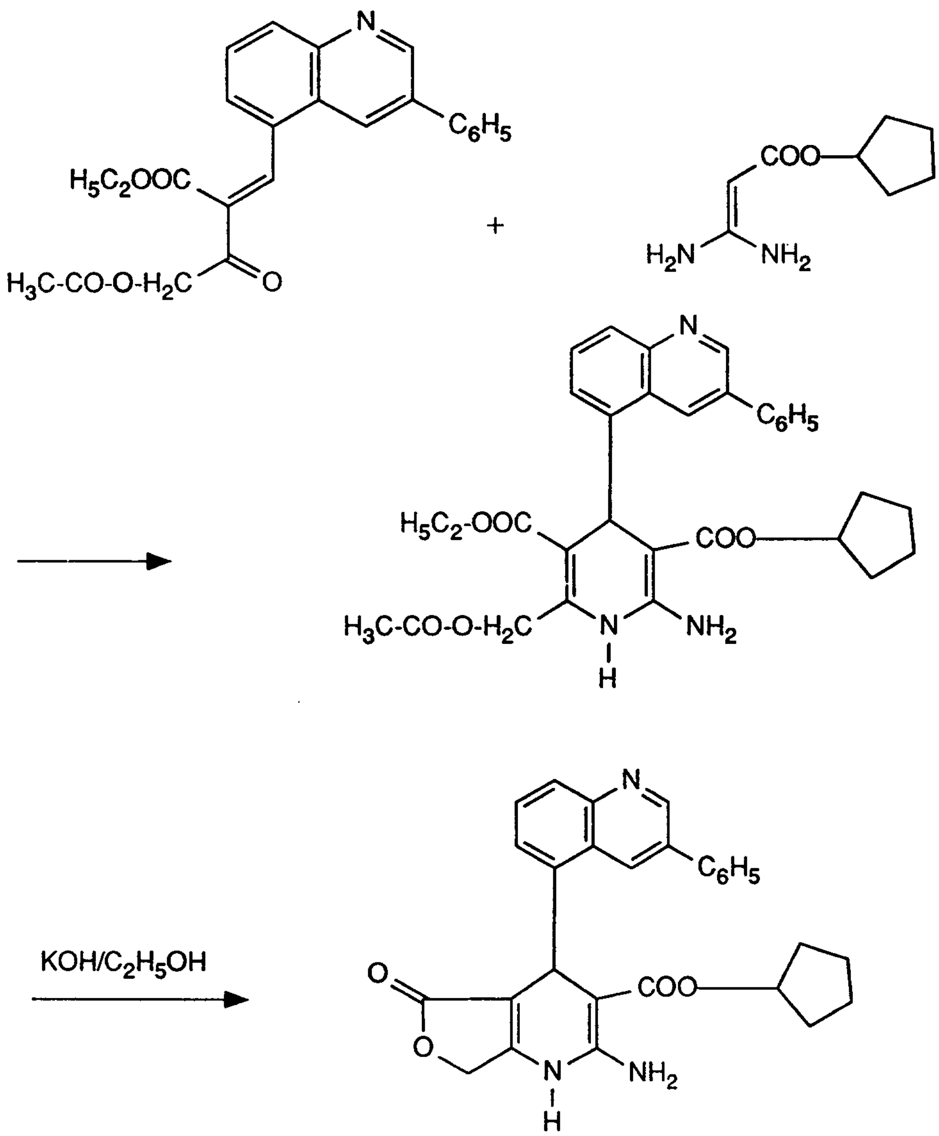


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind in Abhängigkeit von der jeweiligen Verfahrensvariante [A], [B] oder [C] Methanol, Isopropanol, Ethanol und n-Propanol, Acetonitril oder Tetrahydrofuran.

Die Reaktionstemperaturen können in einem größeren Bereich variiert werden. Im allgemeinen arbeitet man zwischen +10 °C und +150 °C, vorzugsweise zwischen +20 °C und +100 °C, insbesondere bei der Siedetemperatur des jeweiligen Lösemittels.

Die Umsetzung kann bei Normaldruck, aber auch bei erhöhtem oder erniedrigtem Druck (z.B. 0,5 bis 3 bar) durchgeführt werden. Im allgemeinen arbeitet man bei Normaldruck.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von

Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Verbindungen der allgemeinen Formel (II) sind teilweise bekannt und können nach üblichen Methoden hergestellt werden, indem man z.B. die entsprechenden Alkyl- oder Hydroxyalkyl-chinoline oxidiert oder die entsprechenden Carboxychinoline reduziert.

Alternativ kann auch 4-Amino-3-hydroxyphthalid, das durch übliche Hydrierung des literaturbekannten 4-Nitro-3-hydroxyphthalid in Anwesenheit eines Katalysators, bevorzugt mit Palladium/Bariumsulfat, erhalten wird, mit Verbindungen der allgemeinen Formel $R^1$-$CH_2$-CHO, die teilweise bekannt sind, zu Verbindungen der allgemeinen Formel (II) über die entsprechenden Carbonsäuren umgesetzt werden.

Die Verbindungen der allgemeinen Formel (III), (IV), (IVa), (V), (VI), (VII) und (VIIa) sind bekannt oder können nach literaturbekannten Methoden hergestellt werden.

Die Verbindungen der allgemeinen Formel (VIII) sind neu, lassen sich aber ebenfalls nach bekannten Methoden herstellen.

Die erfindungsgemäßen Verbindungen der Formel (I) zeigen ein nicht vorhersehbares, wertvolles pharmakologisches Wirkspektrum. Sie beeinflussen die Kontraktionskraft des Herzens und den Tonus der glatten Muskulatur. Vorzugsweise zeigen sie eine positiv inotrope Wirkung. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. als Perfusionsmedium dient eine Krebs-Henseleit-Lösung (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2$EDTA), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert. Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten

gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Beispiel 1

2-Amino-6-methyl-5-nitro-4-(3-phenylchinolin-5-yl)-1,4-dihydropyridin-3-carbonsäureethylester

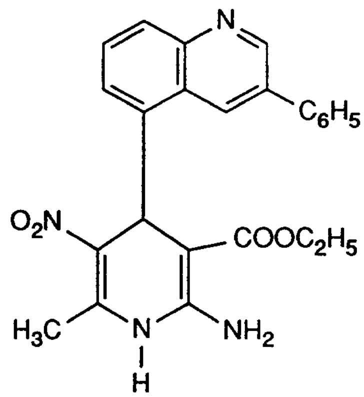

2,33 g (10 mmol) 3-Phenylchinolin-5-carbaldehyd werden in 20 ml Ethanol mit 1,66 g (10 mmol) Amidinoessigsäureethylester-hydrochlorid, 1,8 g (17,5 mmol) Nitroaceton und 820 mg (10 mmol) Natriumacetat versetzt und 30 min zum Rückfluß erhitzt. Die erhaltene dunkelrote Lösung wird abgekühlt und eingeengt. Es wird in Essigester/Wasser gelöst, getrennt, die Essigesterphase wird mit Natriumhydrogencarbonatlösung und Wasser extrahiert, getrocknet und eingeengt. Das erhaltene Gemisch wird über eine Kieselgelsäule mit Toluol/Essigester im Volumenverhältnis 2:1 gereinigt. Die sauberen Fraktionen werden gesammelt und eingeengt. Durch Kristallisation mit Acetonitril erhält man 116 mg gelbe Kristalle vom Schmelzpunkt 252-253 °C.

Beispiel 2

2-Amino-4-(3-phenylchinolin-5-yl)-5-oxo-1,4,5,7-tetrahydrofuro[3,4-b]-pyridin-3-carbonsäureisopropylester

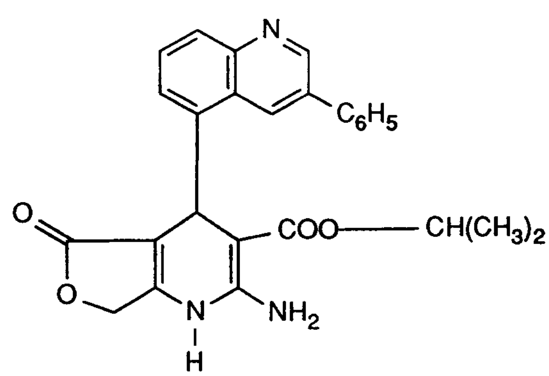

1,2 g (3 mmol) 2-(3-Phenylchinolin-5-yliden)-4-acetoxy-3-oxo-buttersäureethylester werden in 15 ml Isopropanol mit 0,54 g (3 mmol) Amidinoessigsäureisopropylester-hydrochlorid und 0,25 g (3 mmol) Natriumacetat über Nacht zum Rückfluß erhitzt. Es wird abgekühlt und eingeengt. Der erhaltene Rückstand wird mit Wasser angerieben und abgesaugt.

0,53 g der Zwischenverbindung werden in 15 mi Methanol mit 0,25 g Kaliumhydroxid versetzt und 1 Stunde zum Rückfluß erhitzt. Es wird abgekühlt und mit 4,5 ml 1 N Salzsäure neutralisiert. Die ausgefallenen Kristalle werden abgesaugt und mit Methanol gewaschen. Man erhält 290 mg farblose Kristalle vom Schmelzpunkt 199 °C.

Die in den Tabellen 1 und 2 aufgeführten Verbindungen werden in Analogie zu den Vorschriften dei Beispiele 1 und 2 hergestellt:

Tabelle 1:

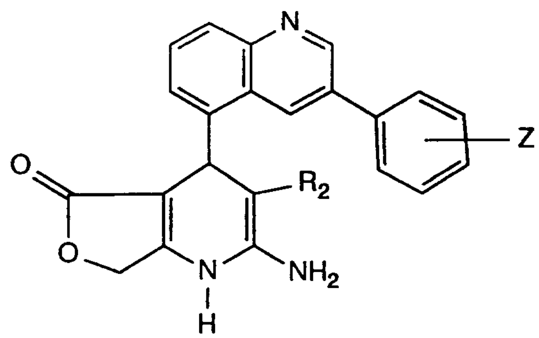

| Bsp.-Nr. | $R^2$ | Z | F°C |
|---|---|---|---|
| 3 | $-CO_2-(CH_2)_2-CH_3$ | H | 187 |
| 4 | $-CO_2-CH_3$ | H | 220 |
| 5 | $-CO_2-CH(CH_3)_2$ | p-F | 205 |
| 6 | $-CO_2C_2H_5$ | p-F | 204 |
| 7 | $-CO_2-(CH_2)_2OCH_3$ | H | 172 |
| 8 | $-CO_2-C_2H_5$ | H | 273 |
| 9 | $-CO_2-(CH_2)_2-OC_2H_5$ | H | 157 |
| 10 | $-CO_2-CH(CH_3)_2$ | H | 199 |
| 11 | $-CO_2CH_3$ | m-$CH_3$ | 203 |
| 12 | $-CO_2(CH_2)_2-OCH_3$ | p-Cl | 160-162 |
| 13 | $-CO_2CH(CH_3)_2$ | p-Cl | 195-197 |
| 14 | $-CO_2CH_3$ | m-$OCH_3$ | 185 |
| 15 | $-CO_2-CH(CH_3)_2$ | m-$OCH_3$ | 168 |
| 16 | $-CO_2CH_3$ | p-Cl | 241-242 |
| 17 | $-CO_2C_2H_5$ | H | 185 |

Tabelle 2:

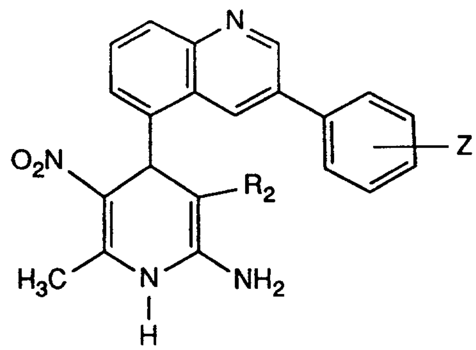

| Bsp.-Nr. | $R^2$ | Z | F°C |
|---|---|---|---|
| 18 | $-CO_2-(CH_2)_2-CH_3$ | H | 229-231 |
| 19 | $-CO_2-(CH_2)_2-OCH_3$ | H | 247 |
| 20 | $-CO_2-CH(CH_3)_2$ | H | 245-246 |

**Patentansprüche**

**1.** 2-Amino-4-chinolin-1,4-dihydropyridine der allgemeinen Formel (I)

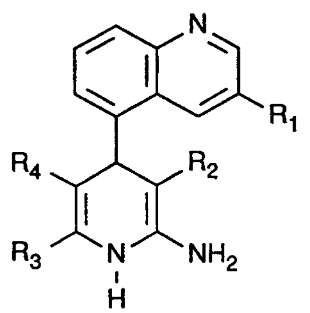

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio, geradkettiges oder verzweigtes Alkoxy oder Alkoxycarbonyl mit jeweils bis zu 8 Kohlenstoffatomen, Carboxy, Amino, Dimethylamino oder Hydroxy oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,
oder
für Thienyl oder Pyridyl steht, die gegebenenfalls durch Halogen substituiert sind,

$R^2$ für eine Gruppe der Formel $-CO-NR^5R^6$ oder $-CO-A-R^7$ steht,
worin

$R^5$ und $R^6$ gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6

bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Carbo- und Hetero-Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^5$ und $R^6$ gemeinsam unter Einbezug des Stickstoffatoms einen 3- bis 8-gliedrigen, gesättigen oder ungesättigten Heterocyclus bilden, der gegebenenfalls durch ein Sauerstoffatom oder durch einen Rest der Formel $S(O)_a$, -CO- oder $-NR^8$ unterbrochen sein kann,

worin

a eine Zahl 0, 1 oder 2 bedeutet,

$R^8$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder

einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

A eine direkte Bindung oder ein Sauerstoffatom bedeutet,

$R^7$ Wasserstoff oder Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_b-$ oder $-NR^9$ unterbrochen ist,

worin

b die oben angegebene Bedeutung von a hat und mit dieser gleich oder verschieden ist,

$R^9$ die oben angegebene Bedeutung von $R^8$ hat und mit dieser gleich oder verschieden ist,
oder der Kohlenwasserstoffrest gegebenenfalls bis zu 3-fach gleich oder verschieden durch Aryliden mit 6 bis 10 Kohlenstoffatomen oder heterocyclische Reste der Formeln

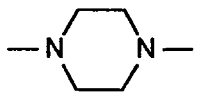 , 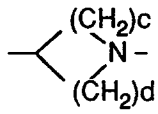 , 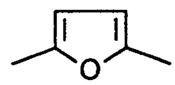 oder 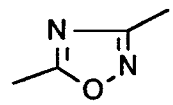

unterbrochen ist,
worin

c und d gleich oder verschieden sind und eine Zahl 1 oder 2 bedeuten,
und worin Aryliden seinerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigten Heterocyclus mit bis zu 3 Heteroatomen aus der Reihe S, N oder O substituiert ist, die ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
durch eine Gruppe der Formel $-CO_2-R^{10}$, $-CONR^{11}R^{12}$ oder $-NR^{13}R^{14}$ substituiert ist,
worin

$R^{10}$ die oben angegebene Bedeutung von $R^8$ hat und mit dieser gleich oder verschieden ist
und

$R^{11}$, $R^{12}$, $R^{13}$ und $R^{14}$ die oben angegebene Bedeutung von $R^5$ und $R^6$ haben und mit diesen gleich oder verschieden sind,

$R^3$ für Wasserstoff oder geradkettiges oder verzweigtes Alkyl mit bis zu 8 Kohlenstoffatomen steht,

$R^4$ für Nitro oder Formyl steht,
oder

$R^3$ und $R^4$ zusammen einen Lactonring der Formel

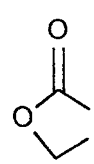

bilden,

und deren Salze.

**2.** Verbindungen der allgemeinen Formel (I) gemäß Anspruch 1, in welcher

$R^1$ für Phenyl steht, das gegebenenfalls bis zu 2-fach gleich oder verschieden durch Halogen, Nitro, Cyano, Trifluormethyl oder durch geradkettiges oder verzweigtes Alkyl oder Alkoxy mit jeweils bis zu 6 Kohlenstoffatomen, Carboxy, Amino, Dimethylamino oder Hydroxy substituiert ist, oder für

Thienyl oder Pyridyl steht, die gegebenenfalls durch Fluor, Chlor oder Brom substituiert sind,

$R^2$ für eine Gruppe der Formel $-CO-NR^5R^6$ oder $-CO-A-R^7$ steht,
worin

$R^5$ und $R^6$ gleich oder verschieden sind und Wasserstoff oder einen geradkettigen, verzweigten, cyclischen, gesättigten oder ungesättigten Kohlenwasserstoffrest mit bis zu 6 Kohlenstoffatomen bedeuten, der gegebenenfalls durch Fluor, Chlor oder durch Phenyl substituiert ist, das seinerseits durch Fluor, Chlor oder durch geradkettiges oder verzweigtes Alkyl, Alkoxy oder Arylthio mit jeweils bis zu 4 Kohlenstoffatomen, Trifluormethyl oder Trifluormethoxy substituiert sein kann, oder
Phenyl bedeuten, das gegebenenfalls durch Fluor, Chlor, Brom, Cyano oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert ist,
oder

$R^5$ und $R^6$ gemeinsam unter Einbezug des Stickstoffatoms einen 5- bis 6-gliedrigen, gesättigen oder ungesättigten Heterocyclus bilden, der gegebenenfalls durch einen Rest der Formel $-NR^8$ unterbrochen sein kann,
worin

$R^8$ Wasserstoff oder Phenyl bedeutet, das gegebenenfalls durch Fluor, Chlor, Brom, Cyano oder durch geradkettiges oder verzweigtes Alkyl, Alkoxy, Alkylthio, Alkoxycarbonyl mit jeweils bis zu 3 Kohlenstoffatomen, Trifluormethyl oder Trifluormethoxy substituiert ist, oder
einen cyclischen, gesättigten oder ungesättigten, geradkettigen oder verzweigten Kohlenwasserstoffrest mit bis zu 6 Kohlenstoffatomen bedeutet, der gegebenenfalls durch Hydroxy, Fluor, Chlor, Brom oder durch Phenyl oder Pyridyl substituiert ist, die ihrerseits bis zu 2-fach gleich oder verschieden durch Fluor, Chlor, Brom, Cyano oder durch geradkettiges oder verzweigtes Alkyl, Alkoxy, Alkylthio, Alkoxycarbonyl mit jeweils bis zu 4 Kohlenstoffatomen, Trifluormethyl oder Trifluormethoxy substituiert sein können,

A eine direkte Bindung oder ein Sauerstoffatom bedeutet,

$R^7$ Wasserstoff oder Phenyl oder Pyridyl bedeutet, die gegebenenfalls durch Fluor, Chlor oder durch geradkettiges oder verzweigtes Alkyl, Alkoxy oder Alkylthio mit jeweils bis zu 4 Kohlenstoffatomen, Trifluormethyl oder Trifluormethoxy substituiert sind, oder
einen cyclischen, geradkettigen oder verzweigten, gesättigten oder ungesättigten Kohlenwasserstoffrest mit bis zu 8 Kohlenstoffatomen bedeutet, der gegebenenfalls bis zu 2-fach gleich oder verschieden durch Sauerstoff oder durch -CO-, -CO-NH-, -O-CO-, -CO-O-, -NH-CO-, $-SO_2-NH-$, $-NH-SO_2-$, $-S(O)_b-$ oder $-NR^9$ unterbrochen ist,
worin

b eine Zahl 0, 1 oder 2 bedeutet,

$R^9$ die oben angegebene Bedeutung Von $R^8$ hat und mit dieser gleich oder verschieden ist,
oder der Kohlenwasserstoffrest gegebenenfalls bis zu 2-fach gleich oder verschieden durch Phenyliden oder heterocyclische Reste der Formeln

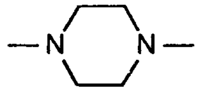 , 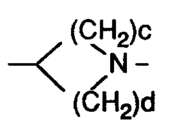 , 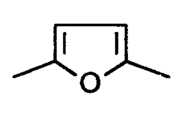 oder 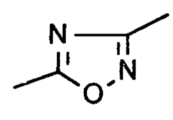

unterbrochen ist,
worin

c und d — gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl, Cyclopentyl, Cyclohexyl, Fluor, Chlor, Brom, Nitro, Cyano, Hydroxy, $-O-NO_2$, geradkettiges oder verzweigtes Alkylthio, Alkoxy oder Acyloxy mit jeweils bis zu 6 Kohlenstoffatomen oder durch Phenyl, Phenoxy, Phenylthio oder Pyridyl substituiert ist, die ihrerseits durch Fluor, Chlor oder durch geradkettiges oder verzweigtes Alkyl, Alkoxy, Alkylthio, Alkoxycarbonyl mit jeweils mit bis zu 4 Kohlenstoffatomen, Trifluormethyl oder Trifluormethoxy substituiert sein können, oder

durch eine Gruppe der Formel $-CO_2-R^{10}$, $-CONR^{11}R^{12}$ oder $-NR^{13}R^{14}$ substituiert ist,

worin

$R^{10}$ — die oben angegebene Bedeutung von $R^8$ hat und mit dieser gleich oder verschieden ist

und

$R^{11}$, $R^{12}$, $R^{13}$ und $R^{14}$ — die oben angegebene Bedeutung von $R^5$ und $R^6$ haben und mit diesen gleich oder verschieden sind,

$R^3$ — für Wasserstoff oder geradkettiges oder verzweigtes Alkyl mit bis zu 4 Kohlenstoffatomen steht,

$R^4$ — für Nitro oder Formyl steht,

oder

$R^3$ und $R^4$ — zusammen einen Lactonring der Formel

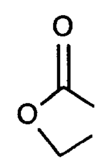

bilden,

und deren Salze.

**3.** Verbindungen der allgemeinen Formel (I) gemäß Anspruch 1, in welcher

$R^1$ — für Phenyl steht, das gegebenenfalls bis zu 2-fach gleich oder verschieden durch Fluor, Chlor, Nitro, Trifluormethyl, Methyl, Ethyl, Methoxy, Ethoxy, Dimethylamino oder Hydroxy substituiert ist, oder für Thienyl oder Pyridyl steht,

$R^2$ — für eine Gruppe der Formel $-CO-NR^5R^6$ oder $-CO-A-R^7$ steht,

worin

$R^5$ und $R^6$ — gleich oder verschieden sind und Wasserstoff oder einen geradkettigen, verzweigten, cyclischen, gesättigten oder ungesättigten Kohlenwasserstoffrest mit bis zu 5 Kohlenstoffatomen bedeuten, der gegebenenfalls durch Fluor, Chlor oder durch Phenyl substituiert ist, das seinerseits durch Fluor, Chlor, Methyl, Methoxy, Methylthio, Trifluormethyl oder Trifluormethoxy substituiert sein kann, oder

Phenyl bedeuten, das gegebenenfalls durch Fluor, Chlor oder durch Methyl, Methoxy, Trifluormethyl oder Trifluormethoxy substituiert ist,

oder

$R^5$ und $R^6$ — gemeinsam unter Einbezug des Stickstoffatoms einen 5- bis 7-gliedrigen, gesättigen oder ungesättigten Heterocyclus bilden, der gegebenenfalls durch ein Sauerstoffatom oder durch einen Rest der Formel $-NR^8$ unterbrochen sein kann,

worin

$R^8$ — Wasserstoff oder Phenyl bedeutet, oder

einen cyclischen, gesättigten oder ungesättigten, geradkettigen oder verzweigten Kohlenwasserstoffrest mit bis zu 4 Kohlenstoffatomen bedeutet,

der gegebenenfalls durch Fluor, Chlor, Brom oder durch Phenyl substituiert ist,

A — eine direkte Bindung oder ein Sauerstoffatom bedeutet,

$R^7$ — Wasserstoff oder Phenyl bedeutet, oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff oder durch -CO-, -CO-NH-, -O-CO-, -CO-O-, -NH-CO-, $-SO_2$-NH-, -NH-$SO_2$-, $-S(O)_b-$ oder $-NR^9$ unterbrochen ist, worin

b — eine Zahl 0, 1 oder 2 bedeutet,

$R^9$ — die oben angegebene Bedeutung von $R^8$ hat und mit dieser gleich oder verschieden ist,

oder der Kohlenwasserstoffrest bis zu 2-fach gleich oder verschieden durch Phenyliden oder heterocyclische Reste der Formeln

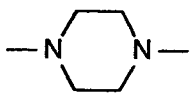 , 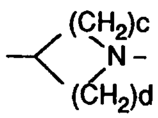 , 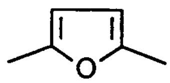 oder 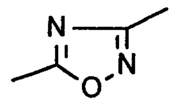

unterbrochen ist,
worin

c und d — gleich oder verschieden sind und eine Zahl 1 oder 2 bedeuten,

und wobei der Kohlenwasserstoffrest gegebenenfalls bis zu 2-fach gleich oder verschieden durch Cyclopropyl, Cyclobutyl. Cyclopentyl, Cyclohexyl, Fluor, Chlor, Nitro, Cyano, Hydroxy, $-O-NO_2$, geradkettiges oder verzweigtes Alkylthio, Alkoxy oder Acyloxy mit jeweils bis zu 4 Kohlenstoffatomen oder durch Phenyl, Phenoxy, Phenylthio oder Pyridyl substituiert ist, die ihrerseits durch Fluor, Chlor oder durch Alkyl, Alkoxy, Alkylthio, Alkoxycarbonyl mit jeweils bis zu 2 Kohlenstoffatomen, Trifluormethyl oder Trifluormethoxy substituiert sein können, oder durch eine Gruppe der Formel $-CO_2-R^{10}$, $-CONR^{11}R^{12}$ oder $-NR^{13}R^{14}$ substituiert ist,
worin

$R^{10}$ — die oben angegebene Bedeutung von $R^8$ hat und mit dieser gleich oder verschieden ist
und

$R^{11}$, $R^{12}$, $R^{13}$ und $R^{14}$ — die oben angegebene Bedeutung von $R^5$ und $R^6$ haben und mit diesen gleich oder verschieden sind,

$R^3$ — für Wasserstoff, Methyl oder Ethyl steht,

$R^4$ — für Nitro oder Formyl steht,
oder

$R^3$ und $R^4$ — zusammen einen Lactonring der Formel

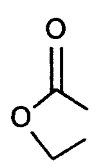

bilden,

und deren Salze.

4. Verbindungen der allgemeinen Formel (I) gemäß Anspruch 1, in welcher

$R^1$ — für Phenyl steht, das gegebenenfalls durch Fluor, Chlor, Methyl oder Methoxy substituiert ist,

$R^2$ für eine Gruppe der Formel -CO-A-$R^7$ steht, worin

A ein Sauerstoffatom bedeutet,

$R^7$ Wasserstoff oder einen cyclischen, geradkettigen oder verzweigten, gesättigten oder ungesättigten Kohlenwasserstoffrest mit bis zu 8 Kohlenstoffatomen bedeutet, der gegebenenfalls durch Sauerstoff, Schwefel, $SO_2$, -CO-NH-, -NH-CO-, -CO-O unterbrochen ist, und der gegebenenfalls durch Cyclopropyl, Cyclopentyl, Cyclohexyl, Fluor, Chlor oder durch Phenyl, Phenoxy oder Phenylthio substituiert ist, wobei die Cyclen ihrerseits durch Fluor, Chlor, Methyl oder Methoxy substituiert sein können,

$R^3$ für Wasserstoff oder Methyl steht,

$R^4$ für Nitro steht,

oder

$R^3$ und $R^4$ zusammen einen Lactonring der Formel

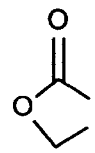

bilden,

und deren Salze.

**5.** Verfahren zur Herstellung von Verbindungen der allgemeinen Formel (I)

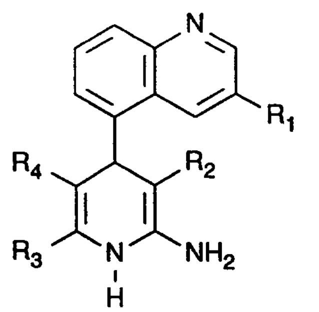

(I)

in welcher

$R^1$ für Aryl mit 6 bis 10 Kohlenstoffatomen steht, das gegebenenfalls bis zu 3-fach gleich oder verschieden durch Halogen, Nitro, Cyano, Trifluormethyl, Trifluormethoxy, Trifluormethylthio, geradkettiges oder verzweigtes Alkoxy oder Alkoxycarbonyl mit jeweils bis zu 8 Kohlenstoffatomen, Carboxy, Amino, Dimethylamino oder Hydroxy oder durch geradkettiges oder verzweigtes Alkyl mit bis zu 8 Kohlenstoffatomen substituiert ist, das seinerseits durch Aryl mit 6 bis 10 Kohlenstoffatomen substituiert sein kann,

oder

für Thienyl oder Pyridyl steht, die gegebenenfalls durch Halogen substituiert sind,

$R^2$ für eine Gruppe der Formel -CO-$NR^5R^6$ oder -CO-A-$R^7$ steht, worin

$R^5$ und $R^6$ gleich oder verschieden sind und Wasserstoff, einen geradkettigen, verzweigten, cyclischen, gesättigten oder ungesättigten Kohlenwasserstoffrest mit bis zu 8 Kohlenstoffatomen bedeuten, der gegebenenfalls durch Halogen, Hydroxy, Cyano oder durch Aryl, Aryloxy, Arylthio mit jeweils 6 bis 10 Kohlenstoffatomen oder durch einen 5- bis 7-gliedrigen, gesättigen oder ungesättigten Heterocyclus mit bis zu 3 Heteroatomen aus der Reihe S, N oder O substituiert ist, wobei die Carbo- und Hetero-Cyclen ihrerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder

Aryl mit 6 bis 10 Kohlenstoffatomen oder einen 5- bis 7-gliedrigen, gesättigten oder ungesättigten Heterocyclus mit bis zu 3 Heteroatomen aus der Reihe S, N oder O bedeuten, die gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind,

oder

$R^5$ und $R^6$ gemeinsam unter Einbezug des Stickstoffatoms einen 3- bis 8-gliedrigen, gesättigten oder ungesättigten Heterocyclus bilden, der gegebenenfalls durch ein Sauerstoffatom oder durch einen Rest der Formel $S(O)_a$, -CO- oder $-NR^8$ unterbrochen sein kann,

worin

a eine Zahl 0, 1 oder 2 bedeutet,

$R^8$ Wasserstoff oder Aryl mit 6 bis 10 Kohlenstoffatomen bedeutet, das gegebenenfalls bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert ist, oder

einen cyclischen, gesättigten oder ungesättigten, geradkettigen oder verzweigten Kohlenwasserstoffrest mit bis zu 8 Kohlenstoffatomen bedeutet, der gegebenenfalls durch Hydroxy, Halogen oder durch Aryl mit 6 bis 10 Kohlenstoffatomen oder einen 5- bis 7-gliedrigen, gesättigten oder ungesättigten Heterocyclus mit 3 Heteroatomen aus der Reihe S, N oder O substituiert ist, die ihrerseits bis zu 2-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,

und der Heterocyclus gegebenenfalls durch geradkettiges oder verzweigtes Alkoxy oder Alkylthio mit jeweils bis zu 4 Kohlenstoffatomen, Halogen, Aryl mit 6 bis 10 Kohlenstoffatomen, einen 5- bis 7-gliedrigen, gesättigten oder ungesättigten Heterocyclus mit bis zu 3 Heteroatomen aus der Reihe S, N oder O oder durch geradkettiges oder verzweigtes Alkyl mit bis zu 4 Kohlenstoffatomen substituiert ist, das gegebenenfalls seinerseits durch Aryl mit 6 bis 10 Kohlenstoffatomen substituiert ist,

A eine direkte Bindung oder ein Sauerstoffatom bedeutet,

$R^7$ Wasserstoff oder Aryl mit 6 bis 10 Kohlenstoffatomen oder einen 5- bis 7-gliedrigen, gesättigten oder ungesättigen Heterocyclus mit bis zu 3 Heteroatomen aus der Reihe S, N oder O bedeutet, die gegebenenfalls bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sind, oder

einen cyclischen, geradkettigen oder verzweigten, gesättigten oder ungesättigten Kohlenwasserstoffrest mit bis zu 10 Kohlenstoffatomen bedeutet, der gegebenenfalls bis zu 3-fach gleich oder verschieden durch Sauerstoff oder durch -CO-, -CO-NH-, -O-CO- -CO-O-, -NH-CO-, $-SO_2-NH-$, $-NH-SO_2-$, $-S(O)_b-$ oder $-NR^9$ unterbrochen ist,

worin

b die oben angegebene Bedeutung von a hat und mit dieser gleich oder verschieden ist,

$R^9$ die oben angegebene Bedeutung von $R^8$ hat und mit dieser gleich oder verschieden ist,

oder der Kohlenwasserstoffrest gegebenenfalls bis zu 3-fach gleich oder verschieden durch Aryliden mit 6 bis 10 Kohlenstoffatomen oder heterocyclische Reste der Formeln

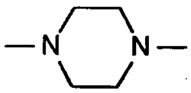 , 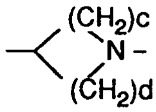 , 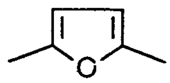 oder 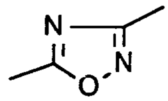

unterbrochen ist,
worin

c und d — gleich oder verschieden sind und eine Zahl 1 oder 2 bedeuten,
und worin Aryliden seinerseits durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können,
und wobei der Kohlenwasserstoffrest gegebenenfalls bis zu 3-fach gleich oder verschieden durch Cycloalkyl mit 3 bis 8 Kohlenstoffatomen, Halogen, Nitro, Cyano, Hydroxy, $-O-NO_2$, geradkettiges oder verzweigtes Alkylthio, Alkoxy oder Acyloxy mit jeweils bis zu 8 Kohlenstoffatomen oder durch Aryl, Aryloxy oder Arylthio mit jeweils 6 bis 10 Kohlenstoffatomen oder durch einen 5- bis 7-gliedrigen, gesättigten oder ungesättigen Heterocyclus mit bis zu 3 Heteroatomen aus der Reihe S, N oder O substituiert ist, die ihrerseits bis zu 3-fach gleich oder verschieden durch Halogen, Cyano oder durch geradkettiges oder verzweigtes Alkyl, Alkoxy, Alkylthio, Alkoxycarbonyl, Halogenalkyl, Halogenalkoxy oder Halogenalkylthio mit jeweils bis zu 4 Kohlenstoffatomen substituiert sein können, oder
durch eine Gruppe der Formel $-CO_2-R^{10}$, $-CONR^{11}R^{12}$ oder $-NR^{13}R^{14}$ substituiert ist,
worin

$R^{10}$ — die oben angegebene Bedeutung von $R^8$ hat und mit dieser gleich oder verschieden ist
und

$R^{11}$, $R^{12}$, $R^{13}$ und $R^{14}$ — die oben angegebene Bedeutung von $R^5$ und $R^6$ haben und mit diesen gleich oder verschieden sind,

$R^3$ — für Wasserstoff oder geradkettiges oder verzweigtes Alkyl mit bis zu 8 Kohlenstoffatomen steht,

$R^4$ — für Nitro oder Formyl steht,
oder

$R^3$ und $R^4$ — zusammen einen Lactonring der Formel

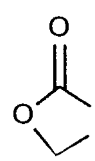

bilden,

und deren Salze,
dadurch gekennzeichnet, daß man

[A] entweder Aldehyde der allgemeinen Formel (II)

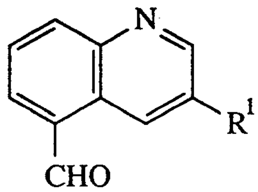

(II)

in welcher

$R^1$ die oben angegebene Bedeutung hat,

direkt mit Verbindungen der allgemeinen Formel (III)

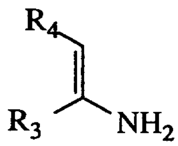

(III)

in welcher

$R^3$ und $R^4$ die oben angegebene Bedeutung haben,

und Verbindungen der tautomeren Formeln (IV) und (IVa)

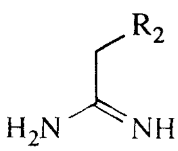

(IV)

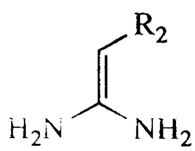

(IVa)

in welcher

$R^2$ die oben angegebene Bedeutung hat,

in inerten Lösemitteln bei Temperaturen zwischen 10 ° C und 150 ° C umsetzt,

oder

[B] Verbindungen der allgemeinen Formel (II) mit Verbindungen der allgemeinen Formel (V)

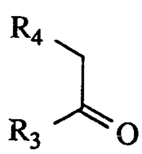

(V)

in welcher

$R^3$ und $R^4$ die oben angegebene Bedeutung haben,

gegebenenfalls nach Isolierung der Ylidenverbindungen der allgemeinen Formel (VI)

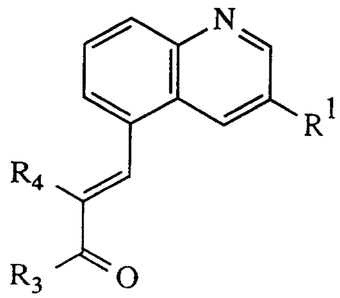

(VI)

in welcher

$R^1$, $R^3$ und $R^4$ die oben angegebene Bedeutung haben,

mit Verbindungen der allgemeinen Formel (VII) bzw. (VIIa)

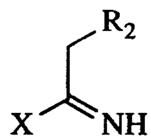

(VII)

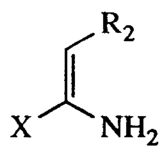

(VIIa)

in welcher

$R^2$ die oben angegebene Bedeutung hat, und

X für die Aminogruppe oder für $C_1$-$C_4$-Alkoxy steht,

gegebenenfalls in Anwesenheit von inerten organischen Lösemitteln bei Temperaturen von 10 °C bis 150 °C umsetzt, wobei für den Fall, daß X für $C_1$-$C_4$-Alkoxy steht, Ammoniumsalze, wie Ammoniumacetat zugegeben werden,

oder im Fall, daß $R^3$ und $R^4$ gemeinsam einen Lactonring bilden

[C] zunächst nach denen unter [A] und [B] aufgeführten Methoden, Verbindungen der allgemeinen Formel (VIII)

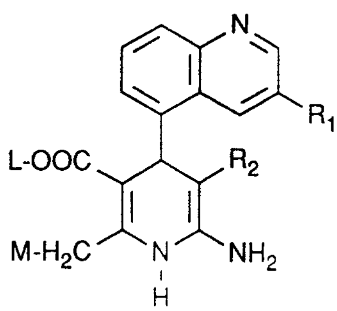

(VIII)

in welcher

$R^1$, $R^2$ und $R^3$ die oben angegebene Bedeutung haben,

L für einen $C_1$-$C_8$-Alkyl-Rest steht

und

M für eine Abgangsgruppe wie beispielsweise Chlor oder Acetoxy steht,

herstellt, und nach bekannten Verfahren einen säure- oder basenkatalysierten Ringschluß anschließt.

**6.** Verbindungen der allgemeinen Formel (I) gemaß Anspruch 1 zur Verwendung bei der Behandlung von Herz-Kreislauferkrankungen.

**7.** Arzneimittel enthaltend mindestens eine Verbindung der allgemeinen Formel (I) gemäß Anspruch 1.

**8.** Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

**9.** Verwendung von Verbindungen der allgemeinen Formel (I) gemäß Anspruch 1 in Arzneimitteln mit positiv inotroper Wirkung.